# EUROPÄISCHE PATENTSCHRIFT

(11) **EP 0 631 491 B1**
(45) Veröffentlichungstag und Bekanntmachung des Hinweises auf die Patenterteilung: **24.03.1999**
(21) Anmeldenummer: 94903716.2
(22) Anmeldetag: 14.01.1994
(51) Int. Cl.: A61B 8/08, A61B 1/00

(54) **VORRICHTUNG ZUM ORTEN UND PUNKTIEREN VON BLUTGEFÄSSEN**
BLOOD VESSEL LOCATING AND PUNCTURING DEVICE
DISPOSTIF PERMETTANT DE LOCALISER ET DE PONCTIONNER DES VAISSEAUX SANGUINS

(30) Priorität: 18.01.1993 CH 127/93; 12.07.1993 CH 2084/93
(43) Veröffentlichungstag der Anmeldung: 04.01.1995
(73) Patentinhaber: DARDEL, Eric Dr., CH-8472 Seuzach (CH)
(72) Erfinder: DARDEL, Eric Dr., CH-8472 Seuzach (CH)
(74) Vertreter: Frei, Alexandra Sarah
(86) Internationale Anmeldenummer: CH9400007
(87) Internationale Veröffentlichungsnummer: WO9415532

(56) Entgegenhaltungen:
- EP-A- 0 104 618
- EP-A- 0 467 291
- EP-A- 0 516 582
- EP-A- 0 540 461
- CH-A- 676 787
- US-A- 4 408 692
- US-A- 4 898 178

## Beschreibung

Der Gegenstand der Erfindung betrifft eine Vorrichtung gemäß Anspruch 1, die mit einem Ultraschall-Doppler-Gerät und einer Punktiernadel in einem sterilen Bereich eine nicht invasive Ortung und eine Punktion von von Blutgefässen erlaubt. Das Ultraschall-Doppler-Gerät ist dabei nicht steril und für wiederholte Anwendungen vorgesehen und wird vor jedem Gebrauch mit Hilfe der sterilen Verpackung steril verpackt.

Es ist bekannt, dass Ultraschallgeräte beim Punktieren von zentralen Venen und von Arterien nützlich sein können. Dies gilt insbesondere für bildgebende Ultrasehallgeräte, auf deren Bildschirme nicht nur ein Schnittbild der untersuchten Körpergegend, sondern auch die Punktiernadel zu sehen ist, was die Punktion erheblich erleichtern kann. Eine solche Methode ist bspw. in New England Journal of Medicine 324:566, February 1991 beschrieben. Ultraschallsonden lassen sich aber nur schwer sterilisieren, weshalb sie vor ihrem Gebrauch in einen sterilen, schlauchartigen Überzug eingepackt werden, wie z.B. in der EP-0'477'581 A1 beschrieben ist. Seitlich an der Ultraschallsonde und über den sterilen Überzug kann ein steriles Koppelstück befestigt werden, welches die Punktiernadel in die Ultraschall-Schnittebene führt, so dass die Punktiernadel auf dem Bildschirm sichtbar ist (wie bspw. in US-4'898'178 beschrieben ist). Solche bildgebende Geräte sind aber nicht sterilisierbar und so gross, dass sie kaum steril eingepackt werden können und deshalb eine eigene Abstellfläche ausserhalb des sterilen Operationsfeldes brauchen. Ein mindestens zwei Meter langes, elektrisches Kabel verbindet dann die Ultraschallsonde mit dem elektronischen Gehäuse, was in den engen räumlichen Verhältnissen von Intensivstationen oder Katheterisationssälen nachteilig ist.

Ebenfalls bekannt sind Ultraschall-Doppler-Geräte, welche nur akustische Signale wiedergeben und deshalb wesentlich kleiner und billiger sein können als die bildgebenden. Bei den meisten dieser Doppler-Geräte ist aber die Punktiernadel mit der Achse des Ultraschallfeldes nahezu identisch. Als Folge liegen die Piezzokristalle der Ultraschallsonde und die Punktiernadel räumlich so eng aneinander, dass eine Kontaminierung der Ultraschallsonde durch das Patientenblut erfolgt. Dies gilt insbesondere für Vorrichtungen, bei welchen die Piezzokristalle sich innerhalb der Nadel oder der Spritze befinden, wie es z.B. in den US-3'556'079, EP-0'260'953 oder US-5'131'394 beschrieben ist. Eine Kontaminierung findet aber auch dann statt, wenn die Ultraschallsonde um die Nadel herum angeordnet ist, wie z.B. in CH-501'410 beschrieben. Die kontaminierten Ultraschallsonden können gassterilisiert werden, aber dieser Vorgang ist zeitraubend und verkürzt zudem die Lebensdauer der teuren Ultraschallsonden erheblich. Teuer ist es auch, die Ultraschallsonde nach einmaligem Gebrauch wegzuwerfen, wie manche Hersteller es empfehlen.

In der EP-0'516'582 A1 wird nun eine Vorrichtung beschrieben, bei welcher eine nicht sterile Ultraschallsonde benützt werden kann, weil sie weder die Punktiernadel, noch die Handschuhe des Arztes, noch die Patientenhaut kontaminieren kann. Auf die nicht sterile Ultraschallsonde wird nämlich ein steriles Koppelstück aufgesteckt, in welchem mit Hilfe eines Spiegels das Ultraschallfeld derart umgelenkt wird, dass die Punktiernadel, die ebenfalls durch das Koppelstück geführt wird, parallel zum Ultraschallfeld und in dessen Zentrum auf die Patientenhaut gerichtet ist Der Abstand zwischen der Punktiernadel und der Ultraschallsonde beträgt dabei mindestens den halben Durchmesser der Ultraschallsonde, so dass diese die Punktiernadel nicht kontaminieren kann. Das Koppelstück muss aber hohe Anforderungen bezüglich der Herstellgenauigkeit erfüllen, weil es die Ultraschallsonde, den Spiegel und die Nadelführung relativ zueinander sehr genau positionieren muss. Zudem muss das Koppelstück luftfrei mit ultraschall-leitendem Gel gefüllt und mit durchstechbaren Elastomer-Membranen abgeschlossen sein, damit das Gel nicht herausfliesst. Diese Merkmale machen das Koppelstück, das als Einwegartikel vorgesehen ist, relativ teuer.

Die meisten bekannten Verfahren zur sterilen Verpackung von nicht sterilen Ultrasthallgeräten oder Sonden bedingen eine Zusammenarbeit einer nicht sterilen mit einer sterilen Person, wie dies beispielsweise in den bereits genannten Schriften EP-0'477'581 A1 und EP-0'516'582 A1 beschrieben ist. Im Dokument EP-A-0540461 ist insbesondere eine Ultraschallsonde mit Nut offenbart, die in eine sterile Kappe mit Führungsrohr einbringbar ist. Das elektrische Kabel zur Verbindung der Sonde mit dem Ultraschallgerät ist dabei allerdings an der sterilen Kappe selber vorgesehen (die Kontaktstellen für die Sonde enthält). Somit ergibt sich aus diesem Gerät keine Notwendigkeit, ein weiteres schlauchförmiges, flexibles Verpackungsstück vorzusehen, durch dessen proximale Öffnung die Sonde und ein Teil des damit verbundenen Kabels einbringbar ist. Gemäss EP-0'540'461 A1 kann eine nicht sterile Person die Verpackung allein übernehmen und die Sonde auf gewohnte Art an die sterile Person übergeben In diesem Falle gehören aber das elektrische Kabel und die beiden elektrischen Stecker, welche die Ultraschallsonde mit der Elektronik verbinden, zum Einwegteil, was diesen teuer macht.

Das Ziel der vorliegenden Erfindung ist nun die Schaffung einer Vorrichtung, mit der in einem sterilen Bereich Blutgefässe nicht-invasiv geortet und punktiert werden können. Bei der erfindungsgemäßen Vorrichtung nach Anspruch 1 kommt insbesondere ein Ultraschall-Doppler-Gerät mit einer entsprechenden Sonde und mit einer Punktiemadel zur Anwendung, wobei das Ultraschall-Doppler-Gerät, insbesondere auch die Sonde, nicht steril zu sein brauchen. Die Verpackung der erfindungsgemäßen Vorrichtung soll als Einwegartikel nicht teuer sein. Die Verpackung soll in üblicher Weise von einer unsterilen Person zusammen mit einer sterilen Person mit den unsterilen Teilen (Ultraschallgerät mit Sonde) unmittelbar vor der Verwendung zusammengebracht werden können. Dabei soll mit Hilfe der Verpackung sowohl die Punktiernadel parallel zum Ultraschallfeld und in dessen Zentrum auf die Patientenhaut richtbar sein als auch soll die Sonde derart nahe an der Patientenhaut positionierbar sein, dass eine Ortung auch ohne Vorlaufstrecke möglich wird. Die Handhabung der Vorrichtung soll dabei mindestens so einfach und problemlos sein wie bei bekannten derartigen Verpackungen. Dieses Ziel wird durch die in den Patentansprüchen definierte Erfindung erreicht.

Das steril verpackte Punktiergerät setzt sich im wesentlichen zusammen aus einem nicht sterilen Ultraschall-Doppler-Gerät, welches akustische und/oder optische Signale senden kann, aus einer erfindungsgemässen sterilen Verpackung und aus einer sterilen Punktiernadel, wobei die Verpackung für den Kontakt mit der Patientenhaut eine Kappe und ein an die Kappe anschliessendes, vorzugsweise flexibles Verpakkungsstück aufweist. Das flexible Verpackungsstück besteht beispielsweise aus einem Stück eines flexiblen Schlauches, das an einem Ende mit der Kappe verschlossen ist und am anderen Ende verschliessbar ist Kurz vor der Benutzung des Punktiergerätes wird das nicht sterile Dopplergerät in diese sterile Verpackung so eingeführt und die Verpackung verschlossen, dass die Aus-senfläche der Verpackung steril bleibt und entsprechend im sterilen Bereich des Operationssaales benützt werden kann. Die Kappe ist derart ausgestaltet, dass durch sie die Sonde des Ultraschall-Doppler-Gerätes in einer definierten Position relativ zu einem in der Kappe integrierten Führungsrohr für die Punktiernadel gehalten wird, derart, dass die Achse des Führungsrohres mit derjenigen des Feldes der Ultraschallsonde im wesentlichen zusammenfällt. Die sterile Verpackung ist beim Gebrauch verschlossen und das Führungsrohr ist durchgehend durch die Kappe angeordnet, sodass die unsterile Ultraschallsonde weder die Punktiernadel, noch den Patienten, noch die Handschuhe des Arztes kontaminieren kann. Nach Gebrauch wird die kostengünstige, sterile Verpackung (Kappe und flexibles Verpackunggsstück) weggeworfen. Weder das Gerät noch die Sonde müssen vor dem nächsten Gebrauch sterilisiert werden.

Das Ultraschall-Doppler-Gerät kann batteriebetrieben sein und ein Taschenformat aufweisen, so dass es keine spezielle Abstellfläche benötigt. Dadurch kann das elektrische Kabel, das die Ultraschallsonde mit dem Rest der Elektronik verbindet, entsprechend kurz sein, was eine leichte Einführung des ganzen unsterilen Gerätes in die sterile Verpackung erlaubt.

Das Ultraschall-Dopplergerät kann aber auch grösser sein, wobei ElektronikGehäuse und Sonde mit einem längeren elektrischen Kabel verbunden sind, derart, dass das Elektronik-Gehäuse ausserhalb des sterilen Feldes gestellt werden kann und folglich nicht steril eingepackt werden muss. Damit die optische Richtungsanzeige sich aber beim Punktieren im Blickfeld des Arztes befindet, ist es dann vorteilhaft, die Richtungsanzeige auf dem Sondengehäuse unterzubringen und nicht auf dem Elektronik-Gehäuse.

Die Kappe kann eine Vorlaufstrecke enthalten, welche die mechanische Ankoppelung zwischen der Ultraschallsonde und der Haut des Patienten erleichtert.

Die als Einwegartikel vorgesehene, erfindungsgemässe Verpackung besteht vorzugsweise aus Kunststoff, wobei einzelne Komponenten wie die Nadelführung auch metallisch sein können. Die Vorlaufstrecke kann aus ultraschallleitendem Kunststoff oder aus wasserhaltigem Gel bestehen

Anhand der nachfolgenden Figuren werden einige Ausführungsbeispiele der erfindungsgemässen Vorrichtung im Detail diskutiert Es zeigen:
- **Fig. 1**: die sterilen Einwegteile einer beispielhaften Ausführungsform der erfindungsgemässen Vorrichtung;
- **Fig.2**: ein Ultraschall-Doppler-Gerät, das in der Vorrichtung gemäss Figur 1 steril verpackt werden kann;
- **Fig. 3a-b**: zwei Phasen beim sterilen Verpacken des nicht sterilen Dopplergerätes nach Fig. 2 mittels der Vorrichtung der Fig. 1;
- **Fig. 4**: eine perspektivische Ansicht einer Kappe für die erfindungemässe Vorrichtung;
- **Fig. 5 und 6**: zwei Ausführungsformen von Kappen mit Vorlaufstrecken im Schnitt;
- **Fig. 7**: eine weitere Ausführungsform der erfindungsgemässen Vorrichtung;
- **Fig. 8**: eine beispielhafte Verbindung zwischen Kappe und flexiblem Verpackungsstück;
- **Fig. 9**: eine weitere Ultraschallsonde;

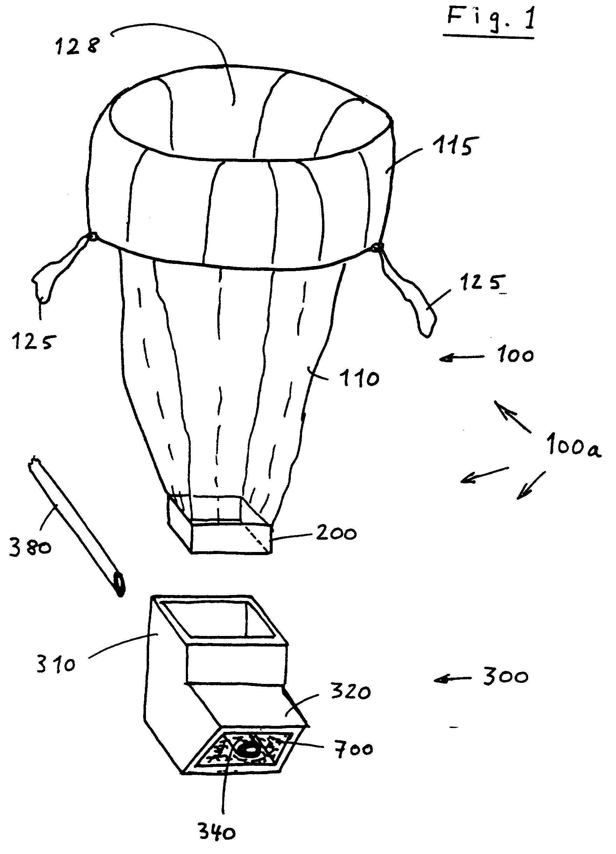

**Fig. 1** zeigt eine beispielhafte Ausführungsform (100a) der Verpackung für die erfindungsgemässe Vorrichtung. Diese besteht im wesentlichen aus einem flexiblen Schlauch 100 (flexibles Verpackungsstück), einer Kappe 300 und einem Rahmen 200 (Verbindung zwischen Kappe und flexiblem Verpackungsstück). Vor der Einführung des Ultraschall-Doppler-Gerätes durch die Öffnung 128 des Schlauches 100 ist dessen oberes Ende 115 zurückgestülpt. Am unteren Ende des Schlauches 100 ist der Rahmen 200 befestigt, welcher eine mechanische Verbindung zwischen dem flexiblen Schlauch 100 und der mindestens teilweise starren Kappe 300 ermöglicht. Wenn die Kappe 300 auf den Rahmen 200 gesteckt ist, bilden sie zusammen mit dem Schlauch 100 einen Hohlraum, der nur eine, beispielsweise mit Bändern 125 verschliessbare Öffnung 128 aufweist. Die Kappe 300 weist einen Sondenführungsteil 310 und einen Vorlaufstreckenteil 320 mit einer Vorlaufstrecke 700 auf. Ein Führungsrohr 340 fuhr eine Punktiernadel 380 durchdringt beide Kappenteile 310 und 320.

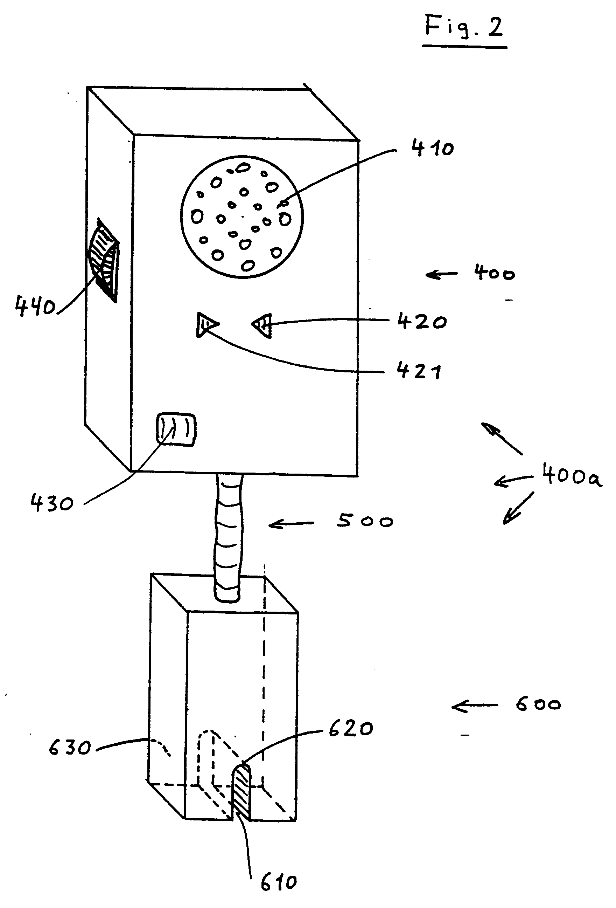

**Fig. 2** zeigt ein Beispiel eines Ultraschall-Dopplergeräts 400a, das in nichtsterilem Zustand in die Vorrichtung gemäss Figur 1 verpackt wird. Es besteht aus einem Elektronik-Gehäuse 400, einem elektrischen Kabel 500 und einer Ultraschallsonde 600. Auf dem Gehäuse 400 befinden sich ein Ein/Aus Schalter 430, ein Lautsprecher 410, ein Drehknopf 440 für die Lautstärkeregulierung, sowie eine optische Anzeige 420, 421 zur Anzeige der Blutstömungsrichtung. Falls die emittierten Ultraschallwellen und das strömende Blut sich weitgehend in die gleiche Richtung bewegen, leuchtet z.B. die optische Anzeige 420, und falls beide Richtungen weitgehend entgegengesetzt sind, so leuchtet die optische Anzeige 421. Die Kombination der akustischen und optischen Signale ist sinnvoll, weil sie die Unterscheidung zwischen Venen und Arterien erleichtern kann.

Die Ultraschallsonde weist in an sich bekannter Weise eine Nut 610 auf, die derart angeordnet ist, dass die Achse eines im innersten Teil 620 der Nut anliegenden Führungsrohres(in der Figur 2 nicht dargestellt) für eine Punktiernadel auf das Zentrum des ausgestrahlten Ultraschallfeldes und parallel zu diesem Feld ausgerichtet ist.

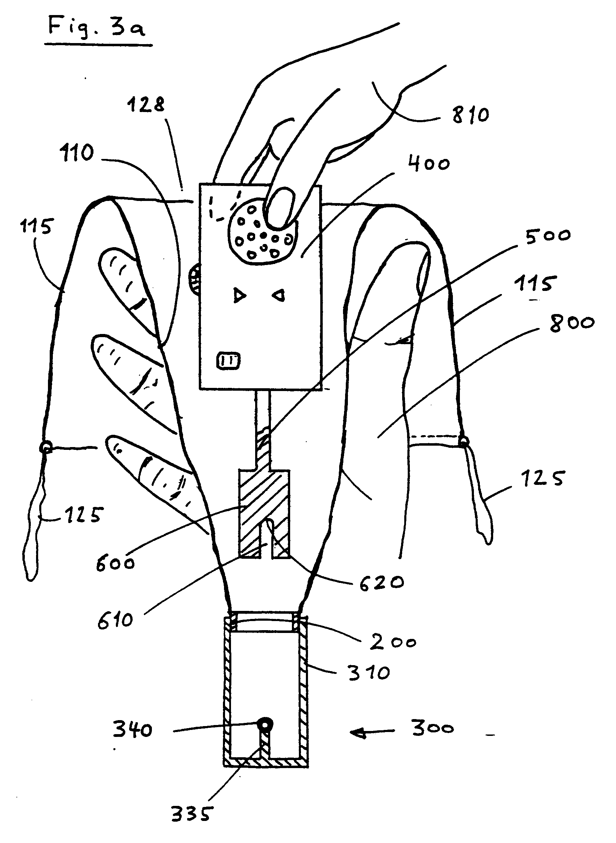

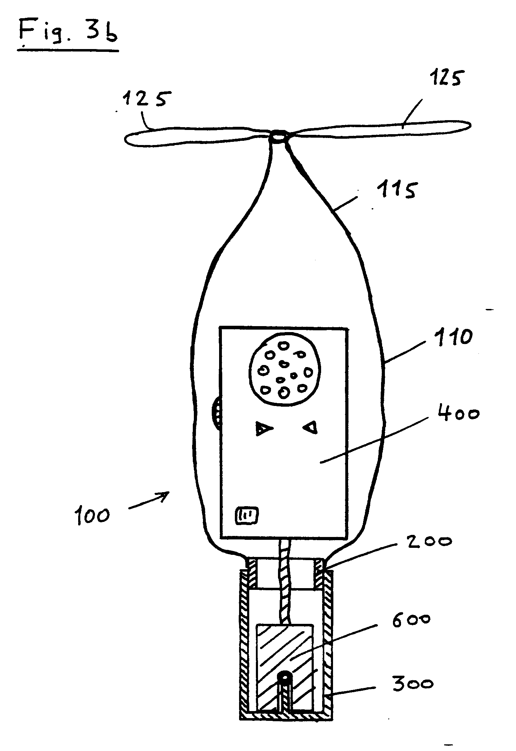

In den **Fig. 3a und 3b** sind zwei Phasen des Einführens des unsterilen Ultraschall-Doppler-Gerätes 400a (Fig. 2) in die erfindungsgemässe Vorrichtung 100a gemäss Fig. 1 dargestellt. Eine sterile Hand 800 wird zwischen den gestülpten Teil 115 und den ungestülpten Teil 110 des schlauchförmigen Verpackungssrückes 100 eingeführt (Fig. 3a), so dass diese Hand 800 von einer Kontamination durch das unsterile Gerät 400a geschützt ist. Die nicht sterile Person führt mit ihrer Hand 810 das nicht sterile Gerät in die Öffnung 128 des Schlauches 100 ein. Mit ihrer zweiten Hand (nicht dargestellt) zieht dann die sterile Person den gestülpten Teil 115 des Schlauches 100 hoch (Fig. 3b) und mit Hilfe der Bänder 125 schliesst sie den Schlauch 100 oben zu. Somit ist das unsterile Gerät 400a in der Vorrichtung 100a verpackt und dadurch für den sterilen Bereich tauglich. Der Schlauch 100 kann selbstverständlich auch mit anderen Mitteln geschlossen werden wie z.B. Minigrips, Klemmvorrichtungen oder Klebstoffen.

Die Ultraschallsonde 600 wird dann durch die sterile Person durch den Schlauch 100 in die Kappe 300 vorgeschoben. Die Sonde 600 wird, vom Sondenführungsteil 310 der Kappe geführt, soweit in die Kappe 300 vorgeschoben, bis das Führungsrohr 340 durch den innersten Teil 620 der Nut 610 verläuft und in der Nut ansteht.

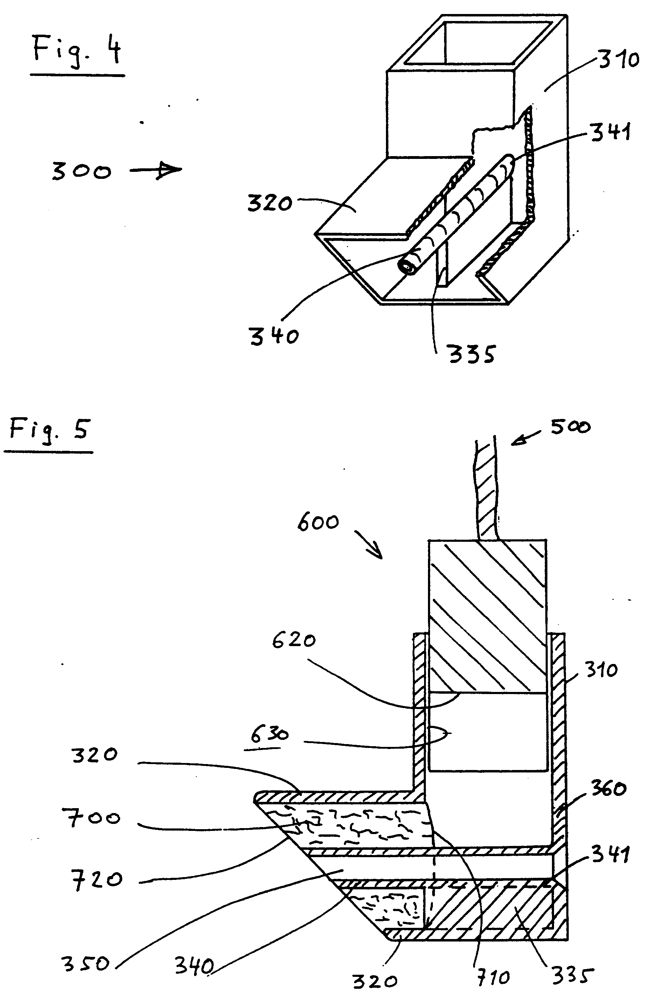

Das Führungsrohr 340 kann, wie auch aus der **Figur 4** ersichtlich ist, an einem Steg 335 befestigt sein. Da der innerste Teil 620 der Nut 610 auf die Richtung des emittierten Feldes ausgerichtet ist, wird eine durch das Führungsrohr 340 eingeführte Punktiernadel (nicht dargestellt) ebenfalls annäherend mit der Ultraschall-Achse zusammenfallen und ein geortetes Gefäss dadurch auch punktieren.

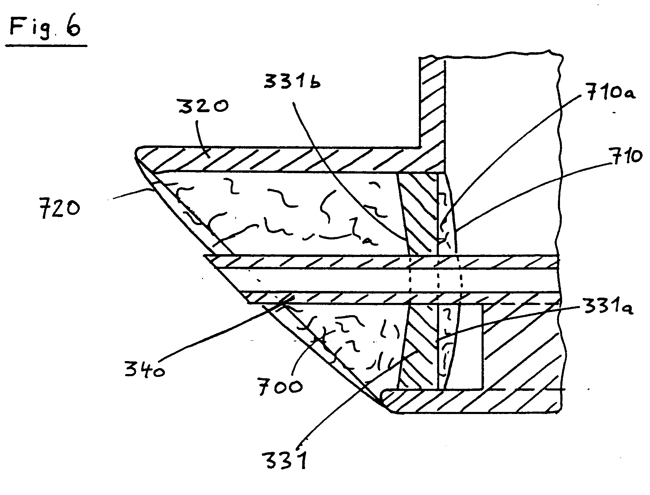

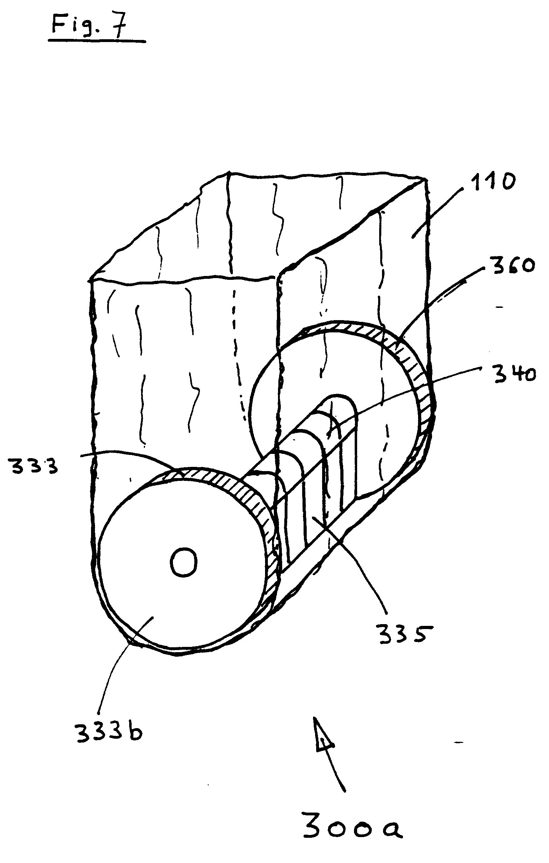

Da mit Hilfe der erfindungsgemässen Vorrichtung die Ultraschallsonde sehr nahe an die Patientenhaut gebracht werden kann, kann auf eine Vorlaufstrecke verzichtet werden (siehe auch Beschreibung im Zusammenhang mit der Figur 7). Ist aber trotzdem eine Vorlaufstrecke vorgesehen, erleichtert diese die mechanische Ankoppelung zwischen der Ultraschallsonde und der Patientenhaut. Figuren 5 und 6 zeigen Ausführungsvarianten der Kappe mit Vorlaufstrecke jeweils im Schnitt.

In der Ausführungsvariante gemäss **Figur 5** besteht die Vorlaufstrecke 700 beispielsweise aus einem formbeständigen, wasserhaltigen Gel, das eine geringe Ultraschalldämpfung aufweist und dank seiner Verformbarkeit eine gute Ankoppelung an die Ultraschallsonde erlaubt. Die sondenseitige Fläche 710 der Vorlaufstrecke 700 ist leicht konvex und wird beim Einschieben der Ultraschallsonde 600 etwas verdrängt, so dass keine Luft zwischen der Stirnfläche 630 der Ultraschallsonde 600 und der sondenseitigen Fläche 710 der Vorlaufstrecke 700 bleibt.

Sondenseitig ist es aber auch möglich eine feste Platte 331 vorzusehen (**Fig. 6**), welche ein Bestandteil der Kappe 300 sein kann. Die Platte 331 trennt die Ultraschallsonde von der Vorlaufstrecke 700 vollständig, so dass letztere durch die Ultraschallsonde nicht verdrängt wird. Die Platte 331 kann aus Kunststoff bestehen, welcher bei einer Dicke von 0,5 bis 2 mm nur eine niedrige Ultraschallabsorbtion aufweist. Um eine gute Ankoppelung zwischen der vorlaufseitigen Stirnfläche der Ultraschallsonde und der Innenfläche 331a der Platte 331 zu gewährleisten sollte mindestens eine der beiden Flächen mit Gel oder Kochsalzlösung vor dem Einfuhren der Sonde in die Kappe benetzt werden. Die Benetzung der Innenfläche 331a der Platte 330 kann während der Herstellung der Kappe 300 erfolgen, indem eine dünne Schicht 710a aus einem ähnlichen Material wie dasjenige der Vorlaufstrecke 700 die Fläche 331a bedeckt.

Die Platte 331 kann auch aus einem Kunststoff bestehen, z.B. aus Plexiglas, der eine grössere Schallausbreitungs-Geschwnidigkeit aufweist, als das wasserhaltige Gel der Vorlaufstrecke 700. Ist dann die Fläche 331b konkav, so hat die Platte 331 eine fokussierende Wirkung auf das Ultraschallfeld. Die Einwegkappe 300 weist somit zusätzlich die Funktion einer auswechselbaren Ultraschall-Linse auf, deren Fokaldistanz auf die Tiefe der zu ortenden Gefässe optimierbar ist. Besteht die Platte aus einem Material mit einer kleineren Schallausbreitungsgeschwindigkeit als das Material der Vorlaufstrecke, wird die Fläche 331b der Platte 331 konvex vorgesehen.

Als Material für die Vorlaufstrecke kommt auch ein fester Kunststoff in Frage, wie z.B. Urethangummi. Solche Kunststoffe weisen zwar eine grössere Ultraschallabsorbtion auf als die wasserhaltigen Gele, dafür sind sie nicht glitschig, so dass für ihre Befestigung keine speziellen Vorkehrungen notwendig sind. Die vorlaufstrecke kann, wie in den Figuren dargestellt in einem Vorlaufstreckenteil 320 der Kappe 300 angeordnet sein. Vorlaufstrecke und entsprechende Kappenwandung können auch als separater Teil auf die Kappe aufgesetzt werden. Ist das Vorlaufstreckenmaterial fest, kann die Vorlaufstrecke mit oder ohne entsprechende Wandung vorgesehen sein.

**Figur 7** zeigt eine beispielhafte Ausführungsform der erfindungsgemässen Vorrichtung ohne Vorlaufstrecke 700. Die Kappe (nur Sondenführungsteil) ist in dieser Ausführungsform reduziert auf das Führungsrohr 340, das zusammen mit einem fakultativen Steg 335 zwei Platten 333 und 360 verbindet. Bei der Anwendung dieser Ausführungsform wird zur Ankoppelung, wie es mit konventionellen Ultraschallsonden üblich ist, eine Gelschicht zwischen der Patientenhaut (nicht dargestellt) und der Vorderflache 333b der vorderen Platte 333 aufgebracht. In dieser Ausführungsform ist das kappenseitige Ende des Verpackungsstückes 110 geschlossen Die reduzierte Kappe 300a liegt dann innerhalb des Verpackungsstuckes, welches mit der vorderen Platte 333 und mit der hinteren Platte 360 beispielsweise verschweisst oder verklebt ist. Die Punktiernadel (nicht dargestellt) kann die dünne Wandung des Verpackungsstückes leicht durchstechen.

Für eine Hautkontaktfläche mit einem Winkel von 30° bis 45° zum Führungsrohr kann die Platte 333 keilförmig ausgestaltet sein. Sie besteht dann vorteilhafterweise aus einem als Vorlaufstrecke geeigneten Kunststoff (z. B. Urethangummi) oder in der Art des bereits beschriebenen Vorlaufstreckenteils der Kappe aus einer im wesentlichen rohrförmigen Wandung (beispielsweise aus Kunststoff), die ein Gel als Vorlaufstrecke umgibt.

Die Befestigung eines schlauchförmigen Verpackungsstuckes 100 an der Kappe 300 kann mit Hilfe eines Rahmens 200 erfolgen (**Fig. 8**), welcher am einen Ende des Verpackungsstückes angeklebt, angeschweisst oder angepresst ist, und über welchen die Kappe 300 geschoben wird. Diese Ausführungsform ist dann vorteilhaft, wenn die Kappe 300 erst und Spital und kurz vor dem Gebrauch mit dem Verpackungsstück 100 verbunden wird, weil die Kappe 300 eine Vorlaufstrecke 700 aus einem wassehaltigen Gel aufweist und deshalb in einer eigenen, wasserdampfdichten Verpackung aufbewahrt wird. Die Kappe 300 kann aber auch bereits bei der Fabrikation am Verpackungsstück 100 befestigt werden, was besonders vorteilhaft ist, wenn die Vorlaufstrecke 700 aus Kunststoff besteht, oder wenn sie fehlt, so dass keine wasserdampfdichte Verpackung nötig ist.

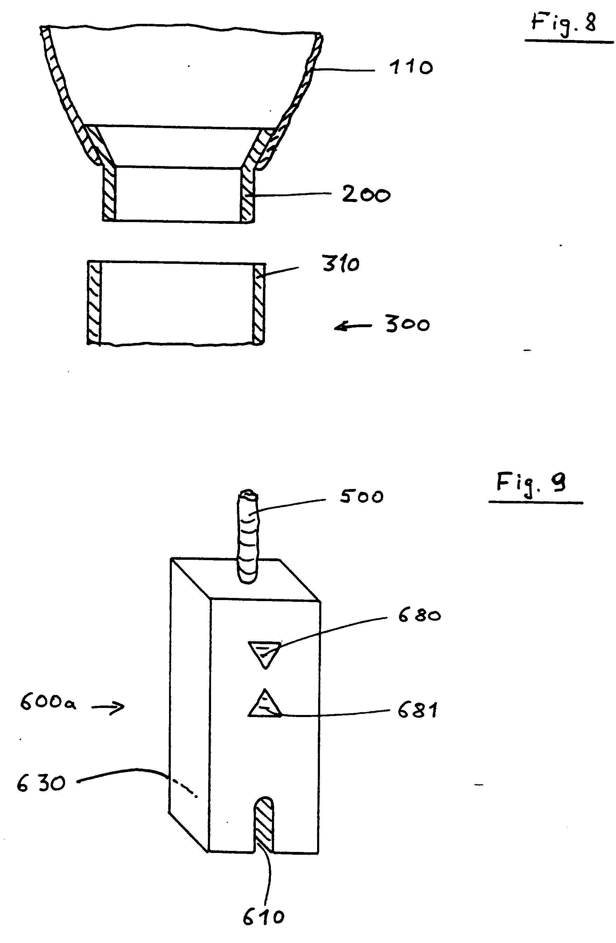

Befindet sich das Elektronik-Gehäuse ausserhalb des sterilen Feldes, muss nur ein Teil des Kabels und die Sonde steril verpackt werden. Damit sich aber auch in diesem Falle die optische Richtungsanzeige während der Punktion im Blickfeld des Arztes befindet, soll sich, wie in **Figur 9** dargestellt, diese Anzeige 680 und 681 auf dem Griff 600a der Ultraschallsonde befinden.

## Patentansprüche

1. Kombination eines nicht sterilen Ultraschall-Doppler-Geräts (400a) zum Orten und Punktieren von Blutgefässen, welches Ultraschall-Doppler-Gerät (400a) eine nicht sterile Sonde (600) mit einer in der Richtung des emittierten Ultraschallfeldes verlaufenden Nut (610), eine nicht sterile elektronische Einheit (400) und ein die Sonde (600) mit der elektronischen Einheit (400) verbindendes, nicht steriles Kabel (500) aufweist, und einer sterilen Verpackung (100a) für das Ultraschall-Doppler-Gerät (400a), wobei die sterile Verpackung (100a) ein schlauchförmiges und flexibles Verpackungsstuck (100) mit einer verschliessbaren Öffnung (128) an seinem proximalen Ende und eine starre Kappe (300) aufweist, wobei die sterile Verpackung (100a) ein in der Nut (610) der Sonde (600) positionierbares Führungsrohr (340) für eine Punktiernadel (380) aufweist, wobei die Kappe (300) vom Führungsrohr (340) durchdrungen ist und derart am distalen Ende des Verpackungsstückes (100) befestigt oder damit verbindbar ist, dass mindestens die Sonde (600) und ein sondenseitiger Teil des Kabels (500) des nicht sterilen Ultraschall-Doppler-Gerätes (400a) durch die verschliessbare Öffnung (128) des Verpackungsstückes (100) in dieses einbringbar sind und dass die Sonde (600) derart in die Kappe (300) führbar ist, dass das Führungsrohr (340) auf dem Grunde der Nut (610) positionierbar ist.

2. Kombination nach Anspruch 1, **dadurch gekennzeichnet,** dass das Führungsrohr (340) langer ist als die Nut (610) der Sonde (600), dass die Kappe (300) derart ausgestaltet ist, dass ein Teil des Führungsrohrs (340) die Nut (610) der in die Kappe geführten Sonde (600) in Richtung eines von der Sonde emittierten Ultraschallfeldes überragt und dass dieser die Nut (610) überragende Teil des Führungsrohres von einer Vorlaufstrecke (700) umgeben ist.

3. Kombination nach Anspruch 2, **dadurch gekennzeichnet,** dass die Vorlaufstrecke (700) in einem Vorlaufstreckenteil (320) der Kappe (300) untergebracht ist.

4. Kombination nach Anspruch 2 oder 3, **dadurch gekennzeichnet,** dass die Vorlaufstrecke (700) aus einem formbeständigen wasserhaltigen Gel besteht, dessen sondenseitige Oberfläche (710) leicht konvex und in der Kappe (300) derart angeordnet ist, dass sie beim Einführen der Sonde (600) in die Kappe (300) von der hautseitigen Fläche (630) der Sonde leicht verdrängt wird.

5. Kombination nach einem der Ansprüche 2 bis 4, **dadurch gekennzeichnet,** dass zwischen der in die Kappe (300) eingeführten Sonde (600) und der Vorlaufstrecke (700) eine vom Führungsrohr (340) durchdrungene Platte (331) angeordnet ist, die zur Bildung einer fokussierenden Linse hautseitig eine konkave oder konvexe Oberfläche (331b) aufweist.

6. Kombination nach einem der Ansprüche 1 bis 5, **dadurch gekennzeichnet,** dass das Führungsrohr (340) mit einem Steg (335) an der Kappe (300) zusätzlich befestigt ist.

7. Kombination nach einem der Ansprüche 1 bis 6, **dadurch gekennzeichnet,** dass das Verpackungsstück (100) an seinem anderen Ende eine weitere Öffnung aufweist, um die ein Rahmen (200) befestigt ist, auf den die Kappe (300) aufsteckbar ist.

## Claims

1. Combination of a non-sterile ultrasonic Doppler apparatus (400a) for locating and puncturing blood vessels, which ultrasonic Doppler apparatus (400a) has a non-sterile probe (600) with a groove (610) running in the direction of the emitted ultrasonic field, a non-sterile electronic unit (400) and a non-sterile cable (500) connecting the probe (600) to the electronic unit (400), and of a sterile package (100a) for the ultrasonic Doppler apparatus (400a), the sterile package (100a) having a tubular and flexible package element (100) with a closable opening (128) at its proximal end and a rigid cap (300), the sterile package (100a) having a guiding tube (340) for a puncturing needle (380), which guiding tube (340) can be positioned in the groove (610) of the probe (600), the cap (300) having the guiding tube (340) passing through it and being secured at or connectable to the distal end of the package element (100) in such a way that at least the probe (600) and a probe-side part of the cable (500) of the non-sterile ultrasonic Doppler apparatus (400a) can be introduced through the closable opening (128) of the package element (100) and into said element, and that the probe (600) can be guided into the cap (300) in such a way that the guiding tube (340) can be positioned at the base of the groove (610).

2. Combination according to Claim 1, characterized in that the guiding tube (340) is longer than the groove (610) of the probe (600), in that the cap (300) is designed in such a way that a part of the guiding tube (340) protrudes beyond the groove (610) of the probe (600), guided into the cap, in the direction of an ultrasonic field emitted by the probe, and in that this part of the guiding tube protruding beyond the groove (610) is surrounded by a foresection (700).

3. Combination according to Claim 2, characterized in that the foresection (700) is accommodated in a foresection part (320) of the cap (300).

4. Combination according to Claim 2 or 3, characterized in that the foresection (700) consists of a shape-stable aqueous gel whose probe-side surface (710) is slightly convex and is arranged in the cap (300) in such a way that it is displaced slightly by the skin-side surface (630) of the probe when the probe (600) is inserted into the cap (300).

5. Combination according to one of Claims 2 to 4, characterized in that arranged between the probe (600), inserted into the cap (300), and the foresection (700) there is a plate (331) through which the guiding tube (340) passes and which has a concave or convex surface (331b) on the skin side for the purpose of forming a focusing lens.

6. Combination according to one of Claims 1 to 5, characterized in that the guiding tube (340) is additionally secured on the cap (300) with a bridge (335).

7. Combination according to one of Claims 1 to 6, characterized in that the package element (100) has, at its other end, a further opening, around which a frame (200) is secured, onto which frame (200) the cap (300) can be attached.

## Revendications

1. Combinaison d'un appareil Doppler à ultrasons (400a) non stérile destiné à localiser et à ponctionner des vaisseaux sanguins, lequel appareil Doppler à ultrasons (400a) comporte une sonde (600) non stérile avec une rainure (610) s'étendant dans la direction du champ d'ultrasons émis, une unité électronique (400) non stérile et un câble (500) non stérile reliant la sonde (600) à l'unité électronique (400), et d'un emballage (100a) stérile destiné à l'appareil Doppler à ultrasons (400a), l'emballage stérile (100a) comportant un élément d'emballage (100) tubulaire et souple avec une ouverture (128) refermable et située à son extrémité proximale, et un embout (300) rigide, l'emballage stérile (100a) comportant un tube de guidage (340) destiné à une aiguille de ponction (380) et pouvant être positionné dans la rainure (610) de la sonde (600), l'embout (300) étant traversé par le tube de guidage (340) et étant fixé ou pouvant être assemblé à l'extrémité distale de l 'élément d'emballage (100) de telle sorte qu'au moins la sonde (600) et une partie du câble (500), côté sonde, de l'appareil Doppler à ultrasons (400a) non stérile puissent être placées à travers l'ouverture (128) refermable de l'élément d'emballage (100) dans celui-ci et que la sonde (600) puisse être guidée dans l'embout (300) de manière à pouvoir positionner le tube de guidage (340) sur le fond de la rainure (610).

2. Combinaison selon la revendication 1, caractérisée en ce que le tube de guidage (340) est plus long que la rainure (610) de la sonde (600), en ce que l'embout (300) est conçu de telle sorte qu'une partie du tube de guidage (340) dépasse la rainure (610) de la sonde (600), guidée dans l'embout (300), dans la direction d'un champ d'ultrasons émis par la sonde et en ce que cette partie, dépassant la rainure (610), du tube de guidage est entourée d'une avancée (700).

3. Combinaison selon la revendication 2, caractérisée en ce que l'avancée (700) est agencée dans une partie d'avancée (320) de l'embout (300).

4. Combinaison selon la revendication 2 ou 3, caractérisée en ce que l'avancée (700) est constituée par un gel, gardant sa forme et contenant de l'eau, dont la surface (710) côté sonde est légèrement convexe et est agencée dans l'embout (300) de telle sorte que, lors de l'introduction de la sonde (600) dans l'embout (300), elle est légèrement repoussée par la surface (630), côté peau, de la sonde.

5. Combinaison selon l'une des revendications 2 à 4, caractérisée en ce que, entre la sonde (600) introduite dans l'embout (300) et l'avancée (700), est agencée une plaque (331) qui est traversée par le tube de guidage (340) et qui a, côté peau, une surface concave ou convexe (331b) pour former une lentille de focalisation.

6. Combinaison selon l'une des revendications 1 à 5, caractérisée en ce que le tube de guidage (340) est aussi fixé par une traverse (335) à l'embout (300).

7. Combinaison selon l'une des revendications 1 à 6, caractérisée en ce que l'élément d'emballage (100) comporte à son autre extrémité une autre ouverture autour de laquelle est fixé un cadre (200) sur lequel l'embout (300) peut être monté.
